## Europäisches Patentamt

## European Patent Office

## Office européen des brevets

(11) Veröffentlichungsnummer: **0 226 770**
**B1**

(12) **EUROPÄISCHE PATENTSCHRIFT**

(45) Veröffentlichungstag der Patentschrift:
07.02.90

(51) Int. Cl.⁴: **C22C 5/04, A61K 6/04**

(21) Anmeldenummer: **86115315.3**

(22) Anmeldetag: **05.11.86**

(54) Edelmetallegierung mit Schmelzintervallen oberhalb 1500o C.

(30) Priorität: **03.12.85 DE 3542641**

(43) Veröffentlichungstag der Anmeldung:
**01.07.87 Patentblatt 87/27**

(45) Bekanntmachung des Hinweises auf die Patenterteilung:
**07.02.90 Patentblatt 90/6**

(84) Benannte Vertragsstaaten:
**AT BE CH DE FR GB IT LI LU NL SE**

(56) Entgegenhaltungen:
**DE-A- 2 703 801**
**US-A- 3 305 816**

**CHEMICAL ABSTRACTS, Band 102, Nr. 24, 17. Juni 1985, Seiten 254-255, Zusammenfassung Nr. 207875j, Columbus, Ohio, US; E.I. RYTVIN et al.: "Effect of thermomechanical treatment on the structure and heat resistance of platinum alloys", & SPLAVY TUGOPLAVKIKH REDK. MET. RAB. VYS. TEMP., [MATER.-VSES. SOVESHCH. FIZ.-KHIM. OSN. SOZDANIYA ZHAROPROCHN. MET. MATER.] 1981 (Pub. 1984) 172-5**

(73) Patentinhaber: **Degussa Aktiengesellschaft, Weissfrauenstrasse 9, D-6000 Frankfurt am Main 1(DE)**

(72) Erfinder: **Groll, Werner, Dr., Kardinal-Döpfner-Strasse 5, D-8757 Karlstein(DE)**
Erfinder: **Steinke, Rude, Grünaustrasse 7, D-6450 Hanau 9(DE)**
Erfinder: **Schiwiora, Harry, Theodor-Heuss-Strasse 37, D-7530 Pforzheim(DE)**

EP 0 226 770 B1

**Beschreibung**

Die Erfindung betrifft angußfähige Edelmetallegierungen mit Schmelzintervallen oberhalb 1500° C für Konstruktionselemente, insbesondere in der Dentaltechnik.

In der modernen Zahntechnik werden eine Vielzahl von unterschiedlichen Konstruktionselementen zur Lösung spezieller Probleme bei der Prothesenversorgung eingesetzt. Geschiebe und Anker dienen z.B. der Verbindung von herausnehmbarem und festsitzendem Zahnersatz. Wurzelstifte werden zur Verankerung von Kronen und Brücken in devitalen Zähnen bzw. auf Zahnstümpfen, die nicht mehr über ausreichend Zahnsubstanz oberhalb des Gingivalsaumes verfügen, verwendet. Die Befestigung der Konstruktionselemente an der Prothese kann durch Löten oder durch direktes Angießen der Dentallegierung an das Konstruktionselement erfolgen. Die Verbindung entsteht in beiden Fällen durch metallurgische Vorgänge, indem zwischen einer flüssigen und einer festen metallischen Phase eine Legierungsbildung stattfindet. Diese kann jedoch auf eine nur wenige Atomlagen ausgedehnte Reaktionszone beschränkt sein. Im Falle der Angußtechnik übernimmt die angegossene Legierung selbst die Rolle des Lotes. Da das angußfähige Konstruktionselement in eine Küvette eingebettet werden muß, kann kein Flußmittel verwendet werden, so daß nur der Einsatz von inoxidablen Legierungen für diese Konstruktionselemente möglich ist. Die Solidustemperaturen dieser angußfähigen Legierungen müßen zudem deutlich höher liegen als die Liquidustemperatur der anzugießenden Legierung, um ein Aufschmelzen der angußfähigen Legierung bzw. eine Verformung des Konstruktionselementes, die dessen Funktion beeinträchtigen würde, während des Gießprozesses zu vermeiden. Die angußfähige Legierung muß außerdem eine hohe Festigkeit und Steifigkeit besitzen, um die Konstruktionselemente grazil gestalten zu können. Zur Herstellung der benötigten Halbzeuge (Draht, Stangen etc.) muß die Legierung außerdem gut umformbar sein.

Ein großer Teil der in der Dentaltechnik eingesetzten Konstruktionselemente wird aus Gold-Basislegierungen mit zirka 60–70 Gew.-% Gold und 30–40 Gew.-% Platingruppenmetallen hergestellt. Diese Legierungen besitzen im ausgehärteten Zustand eine hohe Festigkeit. Ihre Solidustemperatur liegt jedoch mit ca. 1350° C nur wenig oberhalb der Liquidustemperaturen von neuerdings verwendeten höher palladiumhaltigen und goldreduzierten Legierungen bzw. im Bereich der Liquidustemperaturen von Nichtedelmetallegierungen im Dentalsektor. Beim Angießen ist dementsprechend mit einer Beschädigung des Konstruktionselementes zu rechnen.

Die Solidustemperatur eines zweiten, zur Herstellung von Konstruktionselementen verwendeten Legierungstyps mit ca. 80 Gew.-% Platin und 20 Gew.-% Iridium liegt mit ca. 1800°C deutlich höher. Diese Legierungen sind jedoch sehr teuer. Im Bereich der Angußzone bilden sich außerdem häufig Blasen, die zu einer Reduzierung der Festigkeit des Angusses führen.

Es war daher Aufgabe der vorliegenden Erfindung, angußfähige Edelmetallegierungen mit Schmelzintervallen oberhalb von 1500°C für Konstruktionselemente, insbesondere in der Dentaltechnik zu entwickeln, die nicht oxidieren, deren mechanische Festigkeit bei den thermischen Beanspruchungen des Angießens gebräuchlicher Dentallegierungen bzw. des Aufbrennens von Keramik erhalten bleibt bzw. die beim Abkühlen selbständig aushärten, die preiswert sind und bei denen in der Verbindungszone keine Blasen auftreten. Außerdem sollte die Zugfestigkeit der Verbindungszone mindestens der 0,2%-Dehngrenze der angegossenen Legierung entsprechen.

Diese Aufgabe wird erfindungsgemäß dadurch gelöst, daß die Edelmetallegierungen 40 bis 70 Gew.-% Platin, 10 bis 40 Gew.-% Palladium, 5 bis 20 Gew.-% Iridium, 0,5 bis 10 Gew.-% Gold, 0 bis 3 Gew.-% Silber, 0 bis 1 Gew.-% Ruthenium und 0 bis 1 Gew.-% Rhodium enthalten, wobei die Summe der Komponenten 100 Gew.-% ergibt.

Vorzugsweise enthalten sie 50 bis 60 Gew.-% Platin, 20 bis 30 Gew.-% Palladium, 10 bis 17,5 Gew.-% Iridium und 2,5 bis 7,5 Gew.-% Gold, wobei die Summe der Komponenten 100 Gew.-% ergibt.

Legierungen mit weniger als 40 Gew.-% Platin zeigen einen deutlichen Abfall der Festigkeit der Verbindungszone. Gehalte von mehr als 70 Gew.-% Platin sind aus preislichen Erwägungen uninteressant. Legierungen mit mehr als 35 Gew.-% Palladium zeigen ebenfalls einen Abfall der Festigkeit in der Verbindungszone und in der Legierung.

Die Herstellung von reinen Platin-Palladium-Iridium-Legierungen erweist sich als schwierig. Die Gußbolzen, die z.B. zur Weiterverarbeitung zu Stangenmaterial oder Draht benötigt werden, wiesen große Erstarrungslunker in der Mitte der Bolzen auf. Durch Zulegieren von Gold bis zu 10 Gew.-% können diese Herstellprobleme beherrscht werden. Gehalte von mehr als 10 Gew.-% Gold führen zu einer deutlichen Verschlechterung des Umformverhaltens. Es zeigte sich nun überraschenderweise, daß Gold in dem erfindungsgemäßen Konzentrationsbereich die Festigkeits- und Angußeigenschaften deutlich verbessert. Während goldfreie Legierungen mit Iridiumgehalten kleiner 15 Gew.-% während des Angießprozesses bzw. beim Keramikbrand sich deutlich entfestigen, wird dies bei goldhaltigen Legierungen auch mit geringem Iridiumgehalt nicht oder nur abgeschwächt beobachtet. Dies ermöglicht eine weitere Kostenreduzierung durch Einsparung des teueren Iridiums.

Durch Zulegieren von Silber wird eine höhere Festigkeit erreicht, gleichzeitig tritt jedoch eine Versprödung und ein verschlechtertes Umformverhalten auf. Die Silberkonzentration in den angußfähigen Legierungen darf daher 3 Gew.-% nicht übersteigen. Ruthenium bzw. Rhodium bis 1 Gew.-% können zu Lasten des Iridiums der Legierung zugegeben werden.

2

An die erfindungsgemäßen Legierungen können alle handelsüblichen Dentallegierungen angegossen werden, wobei in der Verbindungszone keine Blasenbildung zu beobachten ist. Die Zugfestigkeit von Angüssen lagen stets über der 0,2%-Dehngrenze der jeweils angegossenen Legierung. Der Bruch erfolgte in den meisten Fällen in der angegossenen Legierung bzw. in der angußfähigen Legierung, nur in Ausnahmefällen in der Verbindungszone. Die hohen Solidustemperaturen der erfindungsgemäßen Legierungen, die deutlich höher als 1500°C liegen, garantieren, daß sogar beim Anguß von hochschmelzenden Kobalt-Chrom-Aufbrennlegierungen kein Aufschmelzen bzw. keine Verformung stattfindet.

Der Einsatz dieser Legierungen ist nicht auf die Herstellung von Konstruktionselementen beschränkt, sondern kann sich auch auf andere Teile und andere Einsatzbereiche erstrecken.

In der nachfolgenden Tabelle sind die Zusammensetzungen und die wichtigsten Eigenschaften einiger erfindungsgemäßen Legierungen zusammengestellt.

Tabelle

| Zusammensetzung Gew.-% | | | | | Legierungseigenschaften | | Zugfestigkeit Rm/MPa von Angüssen der Legierungen (nach simuliertem Keramikbrand) | |
|---|---|---|---|---|---|---|---|---|
| Pt | Pd | Ir | Au | Ag | Rp0.2/MPa (nach simuliertem Keramikbrand) | Kalt-Umformbarkeit | Leg. 1 (Rp0.2 $^7$470 MPa) | Leg. 2 (Rp0.2 $^2$575 MPA) |
| 60.0 | 20.0 | 19.5 | 0.5 | – | 570 ± 25 | sehr gut | – | – |
| 47.0 | 35.0 | 17.0 | 1.0 | – | 520 ± 30 | sehr gut | – | – |
| 57.5 | 25.0 | 15.0 | 2.5 | – | 670 ± 40 | sehr gut | 575 ± 55 | 693 ± 50 |
| 57.5 | 25.0 | 12.5 | 5.0 | – | 750 ± 20 | gut | 580 ± 33 | 670 ± 60 |
| 52.5 | 30.0 | 10.0 | 7.5 | – | 661 ± 20 | sehr gut | 550 ± 40 | 650 ± 30 |
| 50.0 | 35.0 | 7.5 | 7.5 | – | 550 ± 20 | sehr gut | 555 ± 7.8 | 580 ± 30 |
| 55.0 | 30.0 | 12.0 | 0.5 | 2.5 | 580 ± 40 | befriedigend | 480 ± 20 | 590 ± 40 |

Leg. 1: Au-Pd-Basis-Aufbrennlegierung; Gießtemperatur 1400°C
Leg. 2: Pd-Basis-Aufbrennlegierung; Gießtemperatur 1450°C

## Patentansprüche

1. Angußfähige Edelmetallegierungen mit Schmelzintervallen oberhalb 1500°C für Konstruktionselemente, insbesondere in der Dentaltechnik, dadurch gekennzeichnet, daß sie 40 bis 70 Gew.-% Platin, 10 bis 40 Gew.-% Palladium, 5 bis 20 Gew.-% Iridium, 0,5 bis 10 Gew.-% Gold, 0 bis 3 Gew.-% Silber, 0 bis 1 Gew.-% Ruthenium und 0 bis 1 Gew.-% Rhodium enthalten, wobei die Summe der Komponenten 100 Gew.-% ergibt.

2. Edelmetallegierungen nach Anspruch 1, dadurch gekennzeichnet, daß sie 50 bis 60 Gew.-% Platin, 20 bis 30 Gew.-% Palladium, 10 bis 17,5 Gew.-% Iridium und 2,5 bis 7,5 Gew.-% Gold enthalten, wobei die Summe der Komponenten 100% ergibt.

## Claims

1. Integrally castable noble metal alloys having melting ranges above 1500°C for constructional elements, particularly in dentistry, characterized in that they contain 40 to 70% by weight platinum, 10 to 40% by weight palladium, 5 to 20% by weight iridium, 0.5 to 10% by weight gold, 0 to 3% by weight silver, 0 to 1% by weight ruthenium and 0 to 1% rhodium, the sum of the components being 100% by weight.

2. Noble metal alloys as claimed in claim 1, characterized in that they contain 50 to 60% by weight platinum, 20 to 30% by weight palladium, 10 to 17.5% by weight iridium and 2.5 to 7.5% by weight gold, the sum of the components being 100% by weight.

## Revendications

1. Alliages de métaux nobles susceptibles d'être coulés, ayant des intervalles de fusion au-dessus de 1500°C pour des éléments de construction, en particulier dans la technique dentaire, caractérisés en ce qu'ils contiennent:
de 40 à 70% en poids de platine, de 10 à 40% en poids de palladium, de 5 à 20% en poids d'irridium, de 0,5 à 10% en poids d'or, de 0 à 3% en poids d'argent, de 0 à 1% en poids de ruthénium et de 0 à 1% en poids de rhodium pour lesquels la somme des composants donne 100% en poids.

2. Alliages de métaux nobles selon la revendication 1, caractérisés en ce qu'ils contiennent: de 50 à 60% en poids de platine, de 20 à 30% en poids de palladium, de 10 à 17,5% en poids d'irridium et de 2,5 à 7,5% en poids d'or pour lesquels la somme des composants donne 100%.